# EUROPÄISCHE PATENTSCHRIFT

(11) **EP 2 544 602 B1**
(45) Veröffentlichungstag und Bekanntmachung des Hinweises auf die Patenterteilung: **14.11.2018**
(21) Anmeldenummer: 11707827.9
(22) Anmeldetag: 04.03.2011
(51) Int. Cl.: A61B 17/221, A61B 17/00, A61B 1/018

(54) **HANDBETÄTIGTES FUNKTIONSSCHLAUCHINSTRUMENT UND BEDIENEINRICHTUNG HIERFÜR**
MANUALLY ACTUATED FUNCTION HOSE INSTRUMENT AND OPERATING DEVICE THEREFOR
INSTRUMENT TUBULAIRE FONCTIONNEL ACTIONNÉ MANUELLEMENT ET DISPOSITIF DE COMMANDE À CET EFFET

(30) Priorität: 09.03.2010 DE 102010010798
(43) Veröffentlichungstag der Anmeldung: 16.01.2013
(73) Patentinhaber: Karl Storz SE & Co. KG, 78532 Tuttlingen (DE)
(72) Erfinder: UIHLEIN, Bernhard, 72581 Dettingen (DE)
(86) Internationale Anmeldenummer: PCT/EP2011/053297
(87) Internationale Veröffentlichungsnummer: WO 2011/110493

(56) Entgegenhaltungen:
- DE-A1- 2 325 404
- DE-B3-102005 018 825
- US-A- 5 779 686
- US-A- 6 015 381
- US-A1- 2004 054 377
- US-A1- 2005 113 862
- US-A1- 2005 182 292
- US-A1- 2007 255 311
- US-A1- 2007 270 640
- US-A1- 2008 242 925

## Beschreibung

Die Erfindung bezieht sich auf ein handbetätigtes Funktionsschlauchinstrument mit einem schlauchförmigen Funktionsteil und einem sich in diesem erstreckenden drahtförmigen Funktionsteil, wobei die beiden Funktionsteile axial relativbeweglich sind, um in einem von einem proximalen Endabschnitt entfernten, distalen Abschnitt eine Nutzfunktion auszuüben, und auf eine zugehörige Bedieneinrichtung. Die Bedieneinrichtung beinhaltet ein erstes Bedienteil, das mit dem proximalen Endabschnitt des drahtförmigen Funktionsteils verbunden ist und einen Bedienkopf am proximalen Ende des drahtförmigen Funktionsteils beinhaltet, und ein zweites Bedienteil, das mit dem proximalen Endabschnitt des schlauchförmigen Funktionsteils verbunden ist und einen mit axialem Abstand vor dem Bedienkopf liegenden Bedienbereich aufweist.

Unter dem Begriff schlauchförmiges Funktionsteil ist dabei vorliegend allgemein ein beliebiges langgestrecktes Schlauch- oder Rohrbauteil zu verstehen, das einen Hohlkanal zur Durchführung des drahtförmigen Funktionsteils aufweist. Unter dem Begriff drahtförmiges Funktionsteil ist vorliegend ein beliebiges langgestrecktes, im Hohlkanal des schlauchförmigen Funktionsteils axial relativbeweglich aufgenommenes Bauteil aus einem massiven oder hohlen Metall- oder Kunststoffmaterial zu verstehen.

Handbetätigte Funktionsschlauchinstrumente dieser Art sind beispielsweise im Bereich der endoskopischen Medizintechnik gebräuchlich, speziell in Form von Steinfangkorbinstrumenten mit entfaltbarem Drahtkorb, um Steine oder dergleichen in Gewebehohlräumen einzufangen, sowie ähnlichen Instrumenten wie Drahtfilterinstrumenten, Drahtschlingeninstrumenten und Fangnetzinstrumenten. Bei diesen genannten Anwendungen befindet sich typischerweise am distalen Ende des drahtförmigen Funktionsteils ein entfaltbares Element, wie der Drahtkorb, ein Drahtfilter, eine Drahtschlinge oder ein Fangnetz, und durch axiales Zurück- und Vorbewegen des drahtförmigen Funktionsteils relativ zum dieses umgebenden schlauchförmigen Funktionsteil wird dieses Element in das distale Ende des schlauchförmigen Funktionsteils unter Zusammenfalten eingezogen bzw. unter Entfalten herausbewegt.

Eine ähnliche Anwendung sind Führungsdrahteinheiten für Katheterinstrumente, wobei es sich in diesem Fall beim drahtförmigen Funktionsteil um einen sogenannten Zugdraht und beim schlauchförmigen Funktionsteil um einen diesen umgebenden Schlauch handelt, der im distalen Bereich mit dem Zugdraht verbunden ist. Durch axiale Relativbewegung von Zugdraht und Schlauch kann ein distaler Abschnitt der Führungsdrahteinheit in einer gewünschten Weise verformt, z.B. gebogen, werden, oder ein geschlitzter distaler Abschnitt des schlauchförmigen Funktionsteils kann dadurch axial verkürzt und auf diese Weise drahtkorbartig aufgebogen werden.

Die Offenlegungsschrift US 2005/0113862 A1 offenbart eine derartige Führungsdrahteinheit, bei welcher der Schlauch im proximalen Endabschnitt einen durch Schlitzung axial elastisch zusammendrückbaren Bereich aufweist und die in ihrem proximalen Endabschnitt eine Bedieneinrichtung beinhaltet. Letztere umfasst ein erstes Bedienteil, das mit einem Bedienkopf auf das proximale Ende des Zugdrahts wirkt, und ein zweites Bedienteil, das mit dem Schlauch vor dessen elastischem Bereich verbunden ist. Das zweite Bedienteil beinhaltet eine Griffhülse mit einer Schraubklemme, durch die sie auf dem Schlauch festgeklemmt werden kann. Der Bedienkopf beinhaltet eine Sacklochbohrung, in die das proximale Ende des drahtförmigen Funktionsteils mit dem dieses umgebenden proximalen Ende des Schlauchs hinter dessen elastischem Bereich eingelegt ist. Am Bedienkopf ist axial in distaler Richtung abstehend eine Führungshülse angeformt, die den proximalen Endabschnitt von drahtförmigem und schlauchförmigem Funktionsteil umgibt und teleskopartig in der Griffhülse axial relativbeweglich geführt ist.

Dokument US 2007/255311 A1 offenbart eine Bedieneinrichtung für ein medizinisches Greifinstrument.

Der Erfindung liegt als technisches Problem die Bereitstellung eines handbetätigten Funktionsschlauchinstruments der eingangs genannten Art, das eine vergleichweise einfache, ergonomische und funktionssichere Einhandbedienung ermöglicht, und einer hierfür geeigneten Bedieneinrichtung zugrunde.
Die Erfindung löst dieses Problem durch die Bereitstellung einer Bedieneinrichtung mit den Merkmalen des Anspruchs 1 und eines medizinisches Steinfangkorbinstruments mit den Merkmalen des Anspruchs 6. Die erfindungsgemäße Bedieneinrichtung umfasst ein Fixierteil, mit dem das zweite Bedienteil axial relativbeweglich gekoppelt ist, und einen Bedienring mit umfangsseitiger Fingeraufnahmemulde als zweites Bedienteil, das unlösbar mit dem schlauchförmigen Funktionsteil verbunden ist. Beide Maßnahmen tragen einzeln oder in Kombination dazu bei, dass sich das Funktionsschlauchinstrument bequem und funktionssicher bedienen lässt, insbesondere durch eine Einhandbedienung, bei welcher der Bedienkopf mit dem Daumen einer Hand betätigt wird und der Bedienring zwischen zwei Fingern gehalten wird, z.B. zwischen Zeige- und Mittelfinger oder zwischen Mittel- und Ringfinger, indem die betreffenden Finger in die Fingeraufnahmemulde des Bedienrings gelegt werden. Die Fingeraufnahmemulde dient als Bedienbereich und verhindert ein unabsichtliches Durchrutschen des Bedienrings im Zwischenraum der beiden ihn haltenden Finger. Die unlösbare Verbindung, z.B. eine Klebe- oder Schweißverbindung, hält den Bedienring sicher am schlauchförmigen Funktionsteil, ohne die axiale Beweglichkeit des drahtförmigen Funktionsteils im schlauchförmigen Funktionsteil zu beinträchtigen. Durch Daumenbetätigung des Bedienkopfs kann sehr leicht die gewünschte axiale Relativbewegung zwischen drahtförmigem und schlauchförmigem Funktionsteil bewirkt werden.
Das Fixierteil ermöglicht eine Fixierung der Bedieneinrichtung an einer externen Komponente, z.B. an einem Endoskop im Fall eines entsprechenden medizinischen Endoskopieinstruments. Das zweite Bedienteil kann gegenüber dem Fixierteil axial relativbewegt werden, z.B. durch entsprechende Betätigung des Bedienrings. So kann beispielsweise im Fall eines in einen Endoskopkanal eingeführten medizinischen Funktionsschlauchinstruments, wie eines Steinfangkorbinstruments oder dgl., das schlauchförmige und mit ihm bei Bedarf synchron auch das drahtförmige Funktionsteil im Endoskopkanal axial vor- oder zurückbewegt werden.

Gemäß der Erfindung ist das erste Bedienteil drehfest mit dem drahtförmigen Funktionsteil verbunden und gegenüber dem zweiten Bedienteil drehbeweglich. Diese Maßnahme ermöglicht es, das drahtförmige Funktionsteil relativ zum schlauchförmigen Funktionsteil zu verdrehen, vorzugsweise frei drehbar ohne Drehwinkelbegrenzung, indem das erste Bedienteil entsprechend gegenüber dem zweiten Bedienteil und damit dem mit diesem unlösbar verbundenen schlauchförmigen Funktionsteil gedreht wird. Auch dies lässt sich noch im Rahmen einer Einhandbedienung ausführen. Alternativ kann dazu der Benutzer seine zweite Hand einsetzen.
In einer Weiterbildung der Erfindung ist das erste Bedienteil durch entsprechend ineinander geführte Führungshülsen teleskopartig am zweiten Bedienteil geführt. Dies ermöglicht eine vorteilhafte und funktionssichere Betätigung der beiden Bedienteile relativ zueinander, um die gewünschte axiale Relativbewegung der beiden Funktionsteile zu bewirken. In einer weiteren Ausgestaltung ist eine zwischen den beiden Bedienteilen in Axialrichtung wirkende Schraubenfeder vorgesehen, die den proximalen Endabschnitt sowohl des drahtförmigen als auch des schlauchförmigen Funktionsteils umgibt und sich einerseits am Bedienkopf und andererseits an der Führungshülse des zweiten Bedienteils abstützt. Damit können die beiden Funktionsteile in einer konstruktiv und funktionell vorteilhaften Weise in eine Endstellung ihrer axialen Relativbeweglichkeit auf Druck oder Zug vorgespannt werden, z.B. im Fall eines medizinischen Steinfangkorbinstruments in eine Ausgangsstellung mit in das schlauchförmige Funktionsteil eingezogenem Fangkörbchen.

Gemäß der Erfindung ist unter Verwendung zweier entsprechend ineinander geführter Führungshülsen eine teleskopartige Führung des zweiten Bedienteils am Fixierteil vorgesehen. Damit kann das zweite Bedienteil und mit ihm das schlauchförmige Funktionsteil funktionssicher axial relativ zum Fixierteil vor- oder zurückbewegt werden, je nach Bedarf mit oder ohne das erste Bedienteil und das drahtförmige Funktionsteil. Auch ist ein zwischen dem Fixierteil und dem zweiten Bedienteil in Axialrichtung wirkendes elastisches Vorspannelement vorgesehen, z.B. in Form einer entsprechenden Schraubenfeder. Dadurch kann das schlauchförmige Funktionsteil in eine gewünschte axiale Endstellung relativ zum Fixierteil auf Druck oder Zug vorgespannt werden.

In einer Weiterbildung der Erfindung weist das Fixierteil ein Anschlussmittel zum lösbaren Anbringen an einem Endoskop auf. Dies ermöglicht eine vorteilhafte Verwendung des handbetätigten Funktionsschlauchinstruments für endoskopische Anwendungen.
In einer Weiterbildung der Erfindung ist die Verbindung des ersten Bedienteils mit dem drahtförmigen Funktionsteil lösbar realisiert. Dadurch kann bei Bedarf das erste Bedienteil vom drahtförmigen Funktionsteil gelöst werden. Dies kann beispielsweise in endoskopischen Anwendungen von Vorteil sein, um das erste Bedienteil zusammen mit dem zweiten Bedienteil und dem schlauchartigen Funktionsteil vom drahtförmigen Funktionsteil proximal abziehen zu können.
Das erfindungsgemäße handbetätigte Funktionsschlauchinstrument weist vorteilhafterweise eine erfindungsgemäße Bedieneinrichtung zur Bewirkung der axialen Relativbewegung von schlauchförmigem und drahtförmigem Funktionsteil zwecks Bewirkung der entsprechenden Nutzfunktion auf. Insbesondere kann es sich gemäß der Erfindung dabei um ein Steinfangkorbinstrument handeln. Nicht gemäß der Erfindung, kann es sich um ein Drahtfilterinstrument, ein Drahtschlingeninstrument oder ein Fangnetzinstrument für endoskopische Anwendungen handeln, wie sie dem Fachmann an sich bekannt sind. Dabei kann die Bedieneinrichtung auf Wunsch auch nachträglich an einem ansonsten herkömmlichen Funktionsschlauchinstrument angebracht werden.
Vorteilhafte Ausführungsformen der Erfindung sind in den Zeichnungen dargestellt und werden nachfolgend beschrieben. Hierbei zeigen:
- Fig. 1: eine Längsschnittansicht einer Bedieneinrichtung für ein handbetätigtes Funktionsschlauchinstrument in einer vorgespannten Ausgangsstellung,
- Fig. 2: eine verkürzte Längsschnittansicht eines medizinischen Steinfangkorbinstruments mit der Bedieneinrichtung von Fig. 1 angebracht an einem Endoskopkanal,
- Fig. 3: die Ansicht des Instruments entsprechend Fig. 2 in einer Funktionsstellung mit entfaltetem Fangkörbchen,
- Fig. 4: eine Detailansicht der Bedieneinrichtung für die Funktionsstellung von Fig. 3,
- Fig. 5: die Ansicht des Instruments entsprechend Fig. 2 in einer vorgeschobenen Ausgangsstellung,
- Fig. 6: eine Detailansicht der Bedieneinrichtung entsprechend Fig. 4 für die vorgeschobene Ausgangsstellung von Fig. 5,
- Fig. 7: die Ansicht des Instruments entsprechend Fig. 5 in Funktionsstellung mit entfaltetem Fangkörbchen,
- Fig. 8: eine Detailansicht der Bedieneinrichtung entsprechend Fig. 6 für die Funktionsstellung von Fig. 7 und
- Fig. 9: eine Längsschnittansicht entsprechend Fig. 1 für eine modifizierte Bedieneinrichtung mit lösbarer Verbindung von Bedienkopf und drahtförmigem Funktionsteil.

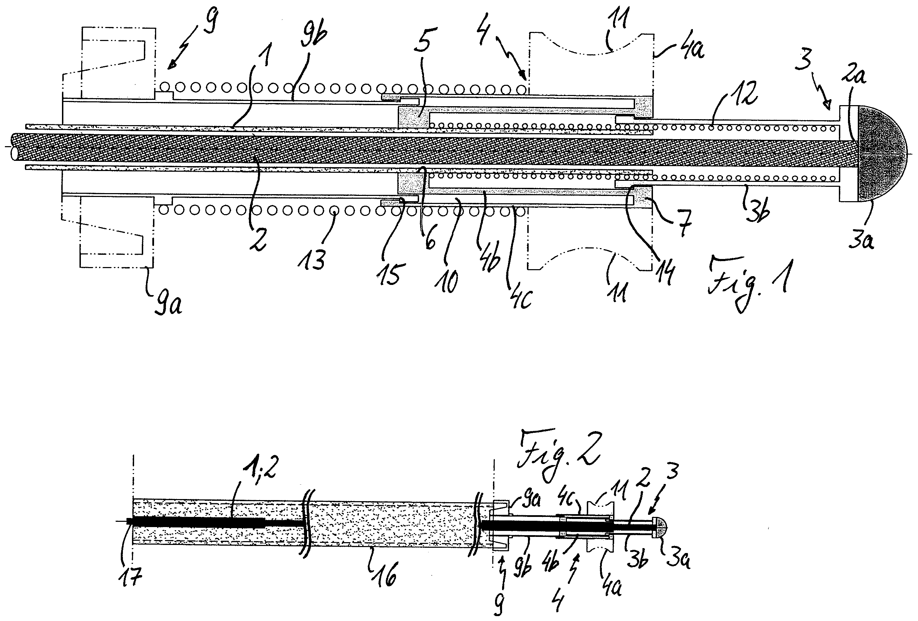

Die in Fig. 1 gezeigte Bedieneinrichtung dient zur handbetätigten Bedienung eines Funktionsschlauchinstruments, das ein schlauchförmiges Funktionsteil 1 und ein sich in diesem axial relativbeweglich erstreckendes drahtförmiges Funktionsteil 2 aufweist, die in Fig. 1 mit ihrem proximalen Endabschnitt dargestellt sind. Die axiale Relativbeweglichkeit des schlauchförmigen Funktionsteils 1, nachfolgend auch kurz als Schlauch bezeichnet, und des drahtförmigen Funktionsteils 2, nachfolgend auch kurz als Funktionsdraht oder Zugdraht bezeichnet, dient zur Bewirkung einer Nutzfunktion in einem vom proximalen Endabschnitt entfernten, distalen Abschnitt des Instruments, wobei es sich nicht zwingend um den distalen Endabschnitt handeln muss, sondern auch um einen zwischen dem proximalen Endabschnitt und einem distalen Endabschnitt liegenden Abschnitt der beiden Funktionsteile 1, 2. Schlauch 1 und Funktionsdraht 2 können aus beliebigen geeigneten, massiven oder hohlen Kunststoff- oder Metallmaterialien bestehen, wie sie für den Einsatzzweck des jeweiligen Funktionsschlauchinstruments dem Fachmann an sich bekannt sind. Je nach Bedarf kann der Schlauch 1 biegeweicher oder biegesteifer als der Funktionsdraht 2 sein.

Die gezeigte Bedieneinrichtung beinhaltet ein bewegungsstarr und unlösbar mit dem Funktionsdraht 2 verbundenes erstes Bedienteil 3 und ein bewegungsstarr und unlösbar mit dem Schlauch 1 verbundenes zweites Bedienteil 4. Das erste Bedienteil 3 beinhaltet einen Bedienkopf 3a, der proximal direkt an ein proximales Ende 2a des Funktionsdrahts 2 anschließt, wobei er mit diesem z.B. durch eine Klebe- oder Schweißverbindung unlösbar verbunden ist. Weiter umfasst das erste Bedienteil 3 eine am Bedienkopf 3a angeformte und von diesem axial in distaler Richtung, d.h. in Fig. 1 nach links, abragende Bedienkopf-Führungshülse 3b. Das zweite Bedienteil 4 weist einen Bedienring 4a und eine Bedienring-Führungshülse 4b auf, die zusammen mit der Bedienkopf-Führungshülse 3b ein Führungsmittel vom Teleskoptyp bildet, durch das die beiden Bedienteile 3, 4 axial relativbeweglich aneinander geführt sind. Außerdem sind die beiden Bedienteile 3, 4 dadurch frei um ihre gemeinsame Längsmittenachse gegeneinander verdrehbar. Dabei ragt die Bedienkopf-Führungshülse (3b) in proximaler Richtung, d.h. nach hinten, aus der Bedienring-Führungshülse (4b) heraus, so dass sich der Bedienkopf (3a) mit axialem Abstand hinter dem Bedienring (4a) befindet.

Die Bedienring-Führungshülse 4b ist an ihrem distalen Stirnende 5 mit einer mittigen Bohrung 6 versehen, durch welche die beiden Funktionsteile 1, 2 hindurchgeführt sind. Am Innenrand ihrer Bohrung 6 ist die Bedienring-Führungshülse 4b fest und unlösbar, z.B. durch eine Klebe- oder Schweißverbindung, mit dem Schlauch 1 verbunden. An ihrem proximalen Stirnende 7 ist die Bedienring-Führungshülse 4b fest mit dem Bedienring 4a verbunden, vorzugsweise dadurch, dass Bedienring 4a und Bedienring-Führungshülse 4b als einstückiges Bauteil gefertigt sind.

Des Weiteren beinhaltet die gezeigte Bedieneinrichtung ein Fixierteil 9, das zur externen Fixierung der gesamten Bedieneinrichtung dient, d.h. zu deren Fixierung an einem externen Bauteil, wie beispielsweise einem Endoskopbauteil im Fall einer medizinischen Anwendung des Funktionsschlauchinstruments in der Endoskopie. Das Fixierteil 9 weist ein Anschlussteil 9a, im gezeigten Beispiel ein herkömmliches Luer-Anschlussteil, und eine Fixierteil-Führungshülse 9b auf, die sich vom Anschlussteil 9a aus in proximaler Richtung axial erstreckt. Korrespondierend dazu weist das zweite Bedienteil 4 eine weitere, äußere Bedienring-Führungshülse 4c auf, welche die andere, innere Bedienring-Führungshülse 4b unter Bildung eines Ringspalts 10 koaxial umgibt. Die Fixierteil-Führungshülse 9b wirkt mit der äußeren Bedienring-Führungshülse 4c unter Bildung eines Führungsmittels vom Teleskoptyp zur axial relativbeweglichen Führung des zweiten Bedienteils 4 am Fixierteil 9 zusammen. Dabei wird die Fixierteil-Führungshülse 9b im Ringspalt 10 zwischen innerer und äußerer Bedienring-Führungshülse 4b, 4c aufgenommen.

Der Bedienring 4a weist, wie gezeigt, an seinem Außenumfang eine umlaufende, halbrunde Ausnehmung 11 auf, die als Fingeraufnahmemulde fungiert und dadurch eine ergonomische und funktionssichere Betätigung der Bedieneinrichtung durch einen Benutzer unterstützt. Dies kann bevorzugt bzw. weitestgehend durch eine Einhandbedienung erfolgen, bei welcher der Benutzer den Bedienring 4a zwischen zwei benachbarten Fingern platziert, die er in einen jeweiligen Bereich der Fingeraufnahmemulde 11 einlegt, z.B. zwischen Zeige- und Mittelfinger oder zwischen Mittel- und Ringfinger, und durch Druck mit dem Daumen auf den Bedienkopf 3a das erste Bedienteil 3 mit dem Funktionsdraht 2 relativ zum zweiten Bedienteil 4 mit dem Schlauch 1 bewegt. Gegenüber Ausführungsbeispielen der Erfindung, bei denen das zweite Bedienteil keine derartige Fingeraufnahmemulde aufweist, ist in den Ausführungsbeispielen mit Fingeraufnahmemulde ein verbesserter Halt des zweiten Bedienteils durch zwei benachbarte Finger einer Hand gegeben. Alternativ zur erwähnten, umfangseitig umlaufenden Fingeraufnahmemulde genügt es in alternativen Ausführungsbeispielen der Erfindung, die Fingeraufnahmemulde durch zwei umfangseitig gegenüberliegende Muldenbereiche eines entsprechend modifizierten Bedienrings zu realisieren, in welche die beiden benachbarten Finger eingreifen können.

Vorzugsweise sind ein oder mehrere elastische Vorspannelemente vorgesehen, um die axial relativbeweglichen Komponenten jeweils in einer axialen Endstellung auf Druck oder Zug vorgespannt zu halten, aus der sie dann durch aktive Nutzerbetätigung herausbewegt werden. Im gezeigten Beispiel von Fig. 1 sind dazu eine erste Schraubendruckfeder 12 zwischen erstem und zweitem Bedienteil 3, 4 und eine zweite Schraubendruckfeder 13 zwischen zweitem Bedienteil 4 und Fixierteil 9 vorgesehen. Die erste Schraubendruckfeder 12 umgibt den proximalen Endabschnitt sowohl des Funktionsdrahts 2 als auch des Schlauchs 1, indem sie sich in einem Ringspalt zwischen dem Schlauch 1 und der Bedienkopf-Führungshülse 3b erstreckt, und stützt sich axial einerseits am distalen Stirnende 5 der inneren Bedienring-Führungshülse 4b und andererseits am Bedienkopf 3a ab. Auf diese Weise spannt die Schraubendruckfeder 12 das erste Bedienteil 3 in eine ausgezogene Stellung seiner Teleskopführung am zweiten Bedienteil 4 auf Druck vor, d.h. in eine Stellung, in welcher der Funktionsdraht 2 gegenüber dem Schlauch 1 zurückgezogen ist. Analog spannt die andere Schraubendruckfeder 13 das zweite Bedienteil 4 in eine ausgezogene Stellung ihrer Teleskopführung am Fixierteil 9 auf Druck vor, wobei sie die beiden zugehörigen Führungshülsen 9b, 4c umgibt und sich einerseits am Anschlussteil 9a und andererseits am Bedienring 4a axial abstützt.

Die Endstellungen, in welche die betreffenden Komponenten der Bedieneinrichtung durch die beiden Schraubendruckfedern 12, 13 vorgespannt werden, sind durch entsprechende Endanschläge der Teleskop-Führungshülsen definiert. Speziell ist die zurückgezogene Endstellung des ersten Bedienteils 3 durch einen korrespondierenden Endanschlag 14 des distalen Endabschnitts der Bedienkopf-Führungshülse 3b am proximalen Endabschnitt der inneren Bedienring-Führungshülse 4b definiert, und die zurückgeschobene Endstellung des zweiten Bedienteils 4 gegenüber dem Fixierteil 9 ist durch einen korrespondierenden Endanschlag 15 des proximalen Endabschnitts der Fixierteil-Führungshülse 9b am distalen Endabschnitt der äußeren Bedienring-Führungshülse 4c definiert.

Nachfolgend wird auf die vorteilhafte Bedienung eines mit der Bedieneinrichtung von Fig. 1 ausgerüsteten, handbetätigten Funktionsschlauchinstruments in Verbindung mit den Fig. 2 bis 8 näher eingegangen, die typische Bedien-/Betriebssituationen veranschaulichen. Dabei wird exemplarisch auf ein medizinisches Steinfangkorbinstrument als handbetätigtes Funktionsschlauchinstrument Bezug genommen. Es versteht sich jedoch, dass die Erfindung in gleicher Weise beliebige andere handbetätigte Funktionsschlauchinstrumente für medizinische und nichtmedizinische Anwendungen umfasst, die mit einer solchen Bedieneinrichtung ausgerüstet sind. Bezüglich medizinischer Anwendungen seien hier insbesondere Drahtfilterinstrumente, Drahtschlingeninstrumente und Fangnetzinstrumente genannt. Weiter wird in den Fig. 2 bis 8 exemplarisch der Fall betrachtet, dass das Funktionsschlauchinstrument bzw. Steinfangkorbinstrument über das Anschlussteil 9a des Fixierteils 9 der Bedieneinrichtung an einem proximalen Ende eines Endoskopkanals 16 fixiert ist, der in den Fig. 2, 3, 5 und 7 jeweils nur verkürzt und schematisch wiedergegeben ist und z.B. von einem Endoskoprohr oder Endoskopschlauch mit proximalem Endoskopanschlussteil gebildet sein kann.

Fig. 2 zeigt das Funktionsschlauchinstrument bzw. Steinfangkorbinstrument in einer Ausgangsstellung im Endoskopkanal 16, die der in Fig.1 gezeigten, vorgespannten Stellung der Bedieneinrichtung entspricht. Dabei sind der Einfachheit halber in den Fig. 2 bis 8 die vorspannenden Federelemente 12, 13 in der Darstellung weggelassen. Ein am distalen Ende des Funktionsdrahts 2 angeordnetes Fangkörbchen 17 befindet sich in der Ausgangsstellung von Fig. 2 in einem zusammengefalteten, in den distalen Endabschnitt des Schlauchs 1 eingezogenen Zustand.

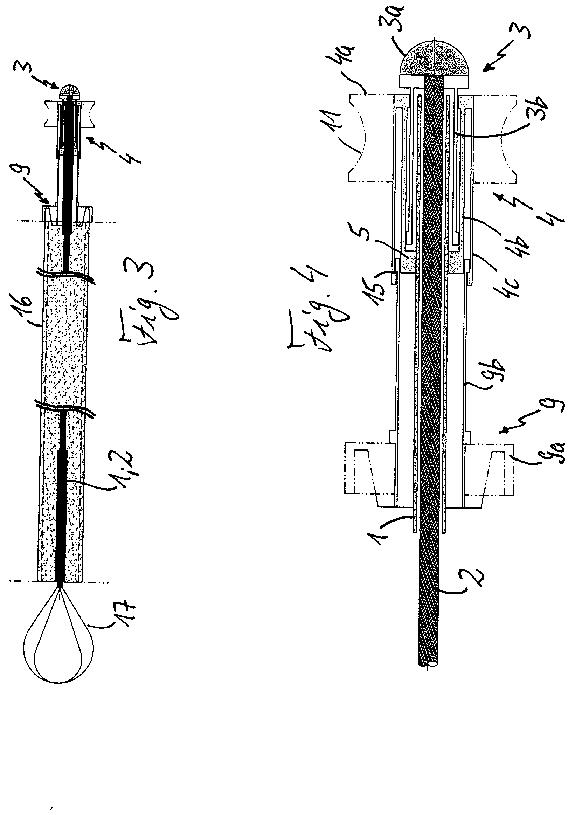

Durch Drücken auf den Bedienkopf 3a, z.B. mit dem Daumen in der oben geschilderten Einhandbedienung bei gleichzeitigem Festhalten des Bedienrings 4a, kann der Funktionsdraht 2 in die in Fig. 3 gezeigte Funktionsstellung relativ zum Schlauch 1 vorgeschoben werden, wodurch sich das Drahtkörbchen 17 aus dem Schlauch 1 herausbewegt und selbsttätig auffaltet. Diese Funktionsendstellung kann z.B. dadurch definiert sein, dass der Bedienkopf 3a gegen den Bedienring 4a bzw. die korrespondierende Bedienring-Führungshülse 4b zur Anlage kommt und/oder die Bedienkopf-Führungshülse 3b mit ihrem distalen Stirnende gegen den distalen Stirnendbereich 5 der korrespondierenden Bedienring-Führungshülse 4b zur Anlage kommt. Dazu entspricht der Hubweg der axialen Relativbeweglichkeit zwischen den beiden Bedienteilen 3, 4 mindestens der aus dem Schlauch 1 herauszubewegenden axialen Länge des Drahtkörbchens 17. In der Detailansicht von Fig. 4 ist die Stellung der Bedieneinrichtung näher zu erkennen, die der Funktionsstellung des Steinfangkorbinstruments gemäß Fig. 3 entspricht.

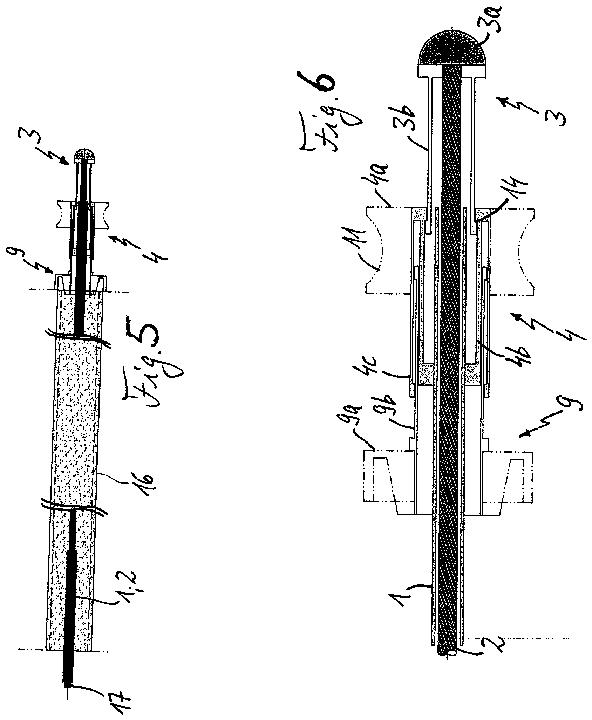

Die Fig. 5 und 6 veranschaulichen eine Situation, in welcher das gesamte Steinfangkorbinstrument, d.h. Schlauch 1 und Zugdraht 2, im Endoskopkanal 16 vorgeschoben werden, beispielsweise um einen weiter vom distalen Ende des Endoskopkanals 16 entfernten Stein einzufangen. Dazu schiebt der Benutzer das zweite Bedienteil 4 am Bedienring 4a distal nach vorn auf das Fixierteil 9 zu. Speziell zeigen die Fig. 5 und 6 das Instrument in einer Stellung, in welcher auf diese Weise die Teleskopführung zwischen zweitem Bedienteil 4 und Fixierteil 9 um etwas mehr als die Hälfte zusammengedrückt wurde. Mit dem zweiten Bedienteil 4 wird dabei auch das erste Bedienteil 3 synchron vorbewegt, das in der entsprechenden Endstellung vorgespannt am zweiten Bedienteil 4 gehalten ist. Schlauch 1 und Zugdraht 2 mit distal in den Schlauch 1 eingezogenem Fangkörbchen 17 werden folglich synchron im Endoskopkanal 16 vorbewegt, bis das distale Instrumentenende eine gewünschte Lage vor dem Endoskopkanal 16 erreicht hat.

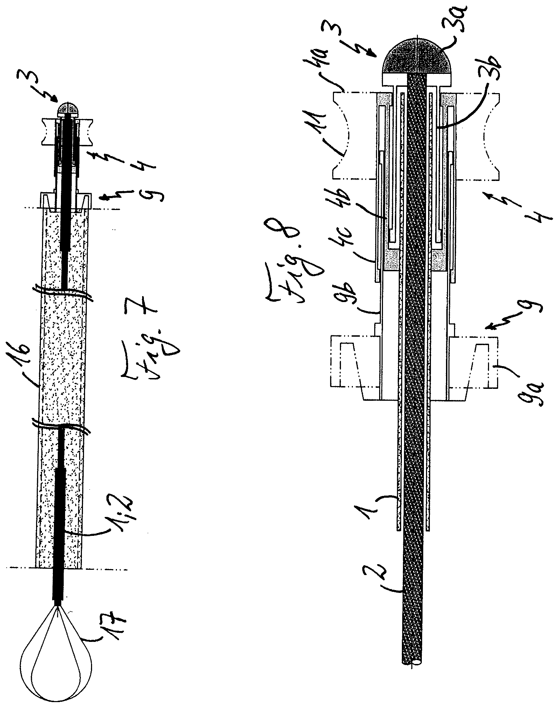

Anschließend kann der Benutzer in dieser Instrumentenstellung das Fangkörbchen 17 entfalten, indem er bei festgehaltenem Bedienring 4a auf den Bedienkopf 3a drückt, bis das Fangkörbchen 17 aus dem distalen Endabschnitt des Schlauchs 1 herausgelangt ist, wie oben zu Fig. 3 erläutert. Die Fig. 7 und 8 zeigen das Instrument in diesem vorgeschobenen Zustand mit selbsttätig aufgefaltetem Fangkörbchen 17. Wie aus Fig. 8 ersichtlich, befindet sich der Bedienkopf 3a in dieser Stellung im Vergleich zur Situation von Fig. 6 in seiner Position benachbart zum Bedienring 4a entsprechend Fig. 4.

In der Stellung mit entfaltetem Fangkörbchen 17 gemäß Fig. 3 oder Fig. 7 kann der Benutzer einen Stein oder einen ähnlichen Gewebepartikel einfangen. Bei Bedarf kann der Benutzer das Fangkörbchen 17 drehen, um das Einfangen des Steins bzw. Partikels zu erleichtern. Dazu braucht er lediglich das erste Bedienteil 3, das bewegungsstarr und damit auch drehfest mit dem Funktionsdraht 2 verbunden ist, an dessen Bedienkopf 3a oder Führungshülse 3b zu drehen. Dies ist ihm im Allgemeinen weiterhin im Rahmen der geschilderten Einhandbedienung möglich. Bei Bedarf kann der Benutzer seine zweite Hand zum Drehen des ersten Bedienteils 3 verwenden. Dabei ist das erste Bedienteil 3, wie auch schon oben erwähnt, frei drehbar am zweiten Bedienteil 4 gehalten. Es sei angemerkt, dass sich bei dieser Drehbewegung der Funktionsdraht 2 im Schlauch 1 dreht, der sich hierbei nicht mitzudrehen braucht. Dies kann mit einer Drehbewegung des Schlauchs 1 im Endoskopkanal 16 einhergehende Probleme, wie erhöhte Reibungseffekte etc., vermeiden.

Sobald der Stein bzw. Partikel eingefangen ist, löst der Benutzer den Druck auf den Bedienkopf 3a, wodurch letzterer mittels der zugehörigen Schraubendruckfeder 12 zurückgedrückt wird. Dadurch wird der Zugdraht 2 relativ zum Schlauch 1 zurückbewegt und das Fangkörbchen 17 partiell in den Schlauch 1 eingezogen, bis es sich so weit zusammengezogen hat, dass es den eingefangenen Stein bzw. Partikel fest umspannt hält. In dieser Stellung kann dann das gesamte Instrument mit dem eingefangenen Stein aus dem betreffenden Gewebekanal herausgezogen werden.

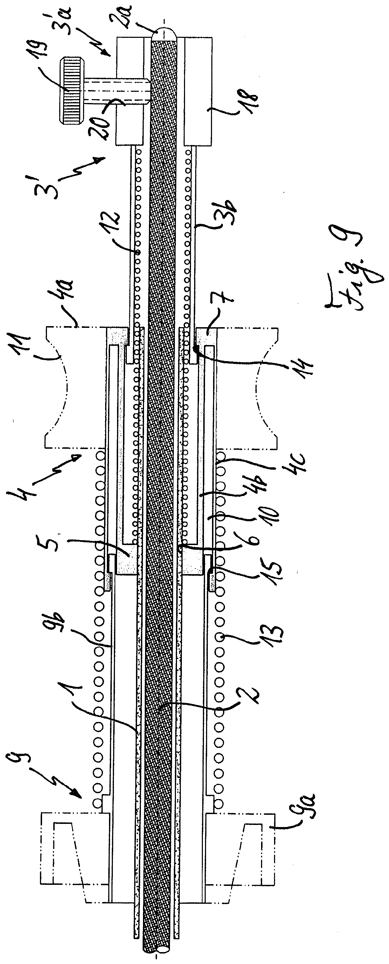

Fig. 9 zeigt eine Variante mit einem modifizierten ersten Bedienteil 3', speziell einem modifizierten Bedienkopf 3'a, wobei die Bedieneinrichtung von Fig. 9 im Übrigen derjenigen der Fig. 1 bis 8 in Aufbau und Funktionsweise entspricht und in Fig. 9 gleiche Bezugszeichen für identische oder funktionell äquivalente Komponenten gewählt sind, so dass insoweit auf die obigen Erläuterungen zur Bedieneinrichtung der Fig. 1 bis 8 verwiesen werden kann. Die Bedieneinrichtung von Fig. 9 unterscheidet sich von derjenigen der Fig. 1 bis 8 darin, dass die Verbindung von Bedienkopf 3'a und Funktionsdraht 2 lösbar realisiert ist. Dazu weist der Bedienkopf 3'a eine Bedienkopfhülse 18 und eine Klemmschraube 19 auf, die in eine radiale Bohrung 20 der Bedienkopfhülse 18 eingeschraubt ist. Die Bedienkopfhülse 18 umgibt den Funktionsdraht 2 in dessen proximalem Endbereich, wobei von ihrem distalen Stirnende die Bedienkopf-Führungshülse 12 abragt und sie mit ihrem proximalen Stirnende im Wesentlichen fluchtend mit dem proximalen Ende 2a des Funktionsdrahts 2 abschließt, das in diesem Beispiel halbkugelförmig gestaltet ist. Durch Festdrehen der Klemmschraube 19 wird der Bedienkopf 3'a am proximalen Endbereich des Funktionsdrahts 2 festgeklemmt. Die Bedienbetätigung des Instruments erfolgt durch Druck z.B. mit dem Daumen auf das proximale Stirnende der Bedienhülse 18, wobei auch das freiliegende halbkugelförmige Ende 2a des Funktionsdrahts 2 als Bedienfläche zur Verfügung steht.

Die lösbare Verbindung von Bedienkopf 3'a und Funktionsdraht 2 hat beispielsweise für den Fall Vorteile, dass mit dem Steinfangkorbinstrument ein Stein im distalen Drahtkörbchen eingefangen wurde, sich jedoch herausstellt, dass er zu groß ist, um ihn durch Herausziehen des Instruments aus dem Endoskopkanal entfernen zu können. In diesem Fall kann bei dem mit der Bedieneinrichtung von Fig. 9 ausgerüsteten Instrument die Klemmverbindung des ersten Bedienteils 3' am Funktionsdraht 2 durch Lösen der Klemmschraube 19 gelöst werden. Nach Lösen der Fixierung der Bedieneinrichtung am Endoskopkanal durch entsprechendes Lösen des Anschlussteils 9a lässt sich dann die gesamte Bedieneinrichtung mit erstem Bedienteil 3', zweitem Bedienteil 4 und Fixierteil 9 samt dem am zweiten Bedienteil 4 fixierten Schlauch 1 proximal nach hinten vom Funktionsdraht 2 abziehen. Die lösbare Verbindung von erstem Bedienteil 3' und Funktionsdraht 2 kann natürlich bei Bedarf auch dazu genutzt werden, nur das erste Bedienteil 3' abzunehmen, wozu dann zusätzlich die Teleskopverbindung der Führungshülsen 4b, 12 zwischen dem ersten Bedienteil 3' und dem zweiten Bedienteil 4 entkoppelt wird.

Die obige, exemplarische Beschreibung der Bedienung eines medizinischen Steinfangkorbinstruments macht deutlich, dass sich durch die erfindungsgemäße Bedieneinrichtung ein entsprechend ausgerüstetes Funktionsschlauchinstrument in sehr vorteilhafter Weise per Hand betätigen lässt, vorzugsweise bzw. weitestgehend mit nur einer Hand. Dazu hält der Benutzer den Bedienring 4a zwischen zwei benachbarten Fingern und betätigt den Bedienkopf 3a mit dem Daumen. Durch axiales Vor- oder Zurückbewegen des Bedienrings 4a relativ zum Fixierteil 9 kann er das gesamte Instrument, d.h. das schlauchförmige und das drahtförmige Funktionsteil synchron, relativ zu einem externen Bauteil, an dem das Instrument mit seinem Fixierteil fixiert ist, vor- bzw. zurückbewegen. Durch Drücken des Bedienkopfs 3a bei festgehaltenem Bedienring 4a kann er eine gewünschte Nutzfunktion im distalen Abschnitt des Instruments ausüben. In jedem Fall benötigt er für den gesamten Bedienvorgang des Instruments keine Bedienhilfe durch eine weitere Person.

Es versteht sich, dass sich die vorteilhaften Wirkungen und Eigenschaften, wie sie oben für die Bedienung eines medizinischen Steinfangkorbinstruments erläutert wurden, in gleicher Weise für die Bedienung anderer handbetätigter Funktionsschlauchinstrumente ergeben, die mit der erfindungsgemäßen Bedieneinrichtung ausgerüstet sind.

## Patentansprüche

1. Bedieneinrichtung für ein medizinisches Steinfangkorbinstrument, das ein schlauchförmiges Funktionsteil (1) und ein sich in diesem erstreckendes drahtförmiges Funktionsteil (2) beinhaltet, wobei die beiden Funktionsteile axial relativbeweglich sind, um in einem von einem proximalen Endabschnitt der Funktionsteile entfernten, distalen Abschnitt eine Nutzfunktion auszuüben, mit
- einem ersten Bedienteil (3), das mit dem proximalen Endabschnitt des drahtförmigen Funktionsteils verbunden ist und einen Bedienkopf (3a) am proximalen Ende des drahtförmigen Funktionsteils beinhaltet und
- einem zweiten Bedienteil (4), das mit dem proximalen Endabschnitt des schlauchförmigen Funktionsteils verbunden ist und einen mit axialem Abstand vor dem Biedenkopf liegenden Bedienbereich (11) aufweist, wobei
- ein Fixierteil (9) vorgesehen ist, das zur externen Fixierung der Bedieneinrichtung dient und mit dem das zweite Bedienteil axial relativbeweglich gekoppelt ist, und
- das zweite Bedienteil unlösbar mit dem proximalen Endabschnitt des schlauchförmigen Funktionsteils verbunden ist und einen Bedienring (4a) mit umfangseitiger Fingeraufnahmemulde (11) als Bedienbereich beinhaltet, wobei
- das zweite Bedienteil teleskopartig am Fixierteil geführt ist, wozu das Fixierteil und das zweite Bedienteil zwei ineinander geführte Führungshülsen (9b, 4c) aufweisen und ein zwischen dem Fixierteil und dem zweiten Bedienteil in Axialrichtung wirkendes elastisches Vorspannelement (13), und wobei das erste Bedienteil drehfest mit dem drahtförmigen Funktionsteil verbunden ist und gegenüber dem zweiten Bedienteil drehbeweglich ist.

2. Bedieneinrichtung nach Anspruch 1, weiter **dadurch gekennzeichnet, dass** das erste Bedienteil teleskopartig am zweiten Bedienteil geführt ist, wozu die beiden Bedienteile zwei ineinander geführte Führungshülsen (3b, 4b) beinhalten.

3. Bedieneinrichtung nach Anspruch 2, weiter **dadurch gekennzeichnet** durch eine zwischen den beiden Bedienteilen in Axialrichtung wirkende Schraubenfeder (12), die den proximalen Endabschnitt des drahtförmigen und des schlauchförmigen Funktionsteils umgibt und sich einerseits am Bedienkopf und andererseits an der Führungshülse des zweiten Bedienteiles abstützt.

4. Bedieneinrichtung nach einem der Anspruche 1 bis 3, weiter **dadurch gekennzeichnet, dass** das Fixierteil ein Anschlussmittel (9a) zum lösbaren Anbringen an einem Endoskop aufweist.

5. Bedieneinrichtung nach einem der Anspruche 1 bis 4, weiter **dadurch gekennzeichnet, dass** das erste Bedienteil (3') lösbar mit dem drahtförmigen Funktionsteil verbunden ist.

6. Medizinisches Steinfangkorbinstrument mit
- einem schlauchförmigen Funktionsteil (1) und einem sich in diesem erstreckenden drahtförmigen Funktionsteil (2), wobei die beiden Funktionsteile axial relativbeweglich sind, um an einem von einem proximalen Endabschnitt entfernten, distalen Abschnitt eine Nutzfunktion auszuüben, und
- einer Bedieneinrichtung, die am proximalen Endabschnitt der Funktionsteile angeordnet ist,
**dadurch gekennzeichnet, dass**
- die Bedienrichtung eine solche nach einem der Anspruch 1 bis 5 ist.

## Claims

1. Operating device for a medical stone-collecting basket instrument, having a hose-shaped functional part (1) and a wire-shaped functional part (2) extending in the latter, wherein the two functional parts are axially movable relative to each other in order to perform a useful function in a distal portion remote from a proximal end portion of the functional parts, with
- a first operating part (3), which is connected to the proximal end portion of the wire-shaped functional part and has an operating head (3a) at the proximal end of the wire-shaped functional part, and
- a second operating part (4), which is connected to the proximal end portion of the hose-shaped functional part and has an operating region (11) lying at an axial distance in front of the operating head,
wherein
- a fixing part (9) is provided, which serves for the external fixing of the operating device and to which the second operating part is coupled in a mutually axially movable manner, and
- the second operating part is connected non-releasably to the proximal end portion of the hose- shaped functional part and has an operating ring (4a) with a circumferential finger recess (11) as operating region,
wherein
- the second operating part is guided telescopically on the fixing part, for which purpose the fixing part and the second operating part have two guide sleeves (9b, 4c) guided one inside the other, and an elastic pretensioning element (13), which acts in the axial direction between the fixing part and the second operating part, and wherein
the first operating part is connected to the wire-shaped functional part for conjoint rotation therewith and is movable in rotation with respect to the second operating part.

2. Operating device according to Claim 1, further **characterized in that** the first operating part is guided telescopically on the second operating part, for which purpose the two operating parts have two guide sleeves (3b, 4b) guided one inside the other.

3. Operating device according to Claim 2, further **characterized by** a helical spring (12), which acts in the axial direction between the two operating parts and which surrounds the proximal end portion of the wire-shaped functional part and of the hose-shaped functional part and bears at one end on the operating head and at the other end on the guide sleeve of the second operating part.

4. Operating device according to one of Claims 1 to 3, further **characterized in that** the fixing part has an attachment means (9a) for releasable mounting on an endoscope.

5. Operating device according to one of Claims 1 to 4, further **characterized in that** the first operating part (3') is connected releasably to the wire-shaped functional part.

6. Medical stone-collecting basket instrument with
- a hose-shaped functional part (1) and a wire-shaped functional part (2) extending in the latter, wherein the two functional parts are axially movable relative to each other in order to perform a useful function at a distal portion remote from a proximal end portion, and
- an operating device, which is arranged on the proximal end portion of the functional parts, **characterized in that**
- the operating device is an operating device according to one of Claims 1 to 5.

## Revendications

1. Dispositif de commande pour un instrument médical de panier de collecte de calculs, qui contient une partie fonctionnelle (1) en forme de tuyau et une partie fonctionnelle (2) en forme de fil s'étendant dans celle-ci, les deux parties fonctionnelles pouvant être déplacées axialement l'une par rapport à l'autre afin d'exercer une fonction utilitaire dans une portion distale éloignée d'une portion d'extrémité proximale des parties fonctionnelles, comprenant
- une première partie de commande (3) qui est connectée à la portion d'extrémité proximale de la partie fonctionnelle en forme de fil et qui contient une tête de commande (3a) au niveau de l'extrémité proximale de la partie fonctionnelle en forme de fil et
- une deuxième partie de commande (4) qui est connectée à la portion d'extrémité proximale de la partie fonctionnelle en forme de tuyau et qui présente une région de commande (11) située à distance axiale de la tête de commande,
- une partie de fixation (9) étant prévue, laquelle sert à la fixation extérieure du dispositif de commande et relativement à laquelle la deuxième partie de commande est accouplée de manière déplaçable axialement, et
- la deuxième partie de commande étant connectée de manière inamovible à la portion d'extrémité proximale de la en forme de tuyau et contenant une bague de commande (4a) avec un creux de réception pour les doigts du côté périphérique (11) servant de région de commande,
- la deuxième partie de commande étant guidée de manière télescopique sur la partie de fixation, ce pourquoi la partie de fixation et la deuxième partie de commande présentent deux douilles de guidage (9b, 4c) guidées l'une dans l'autre et un élément de précontrainte élastique (13) agissant dans la direction axiale entre la partie de fixation et deuxième partie de commande, et
la première partie de commande étant connectée de manière solidaire en rotation à la partie fonctionnelle en forme de fil et pouvant être déplacée en rotation par rapport à la deuxième partie de commande.

2. Dispositif selon la revendication 1, **caractérisée en outre en ce que** la première partie de commande est guidée de manière télescopique sur la deuxième partie de commande, ce pourquoi les deux parties de commande contiennent deux douilles de guidage (3b, 4b) guidées l'une dans l'autre.

3. Dispositif de commande selon la revendication 2, **caractérisé en outre par** un ressort hélicoïdal (12) agissant dans la direction axiale entre les deux parties de commande, qui entoure la portion d'extrémité proximale de la partie fonctionnelle en forme de fil et de la partie fonctionnelle en forme de tuyau et qui s'appuie d'une part sur la tête de commande et d'autre part sur la douille de guidage de la deuxième partie de commande.

4. Dispositif de commande selon l'une quelconque des revendications 1 à 3, **caractérisé en outre en ce que** la partie de fixation présente un moyen de raccord (9a) pour le montage amovible sur un endoscope.

5. Dispositif de commande selon l'une quelconque des revendications 1 à 4, **caractérisé en outre en ce que** la première partie de commande (3') est connectée de manière amovible à la partie fonctionnelle en forme de fil.

6. Instrument médical de panier de collecte de calculs, comprenant
- une partie fonctionnelle (1) en forme de tuyau et une partie fonctionnelle (2) en forme de fil s'étendant dans celle-ci, les deux parties fonctionnelles pouvant être déplacées axialement l'une par rapport à l'autre afin d'exercer une fonction utilitaire sur une portion distale éloignée d'une portion d'extrémité proximale, et
- un dispositif de commande qui est disposé au niveau de la portion d'extrémité proximale des parties fonctionnelles,
**caractérisé en ce que**
- le dispositif de commande est un dispositif selon l'une quelconque des revendications 1 à 5.
